# EUROPEAN PATENT APPLICATION

(11) **EP 4 744 590 A1**
(43) Date of publication of application: **20.05.2026**
(21) Application number: 24213214.0
(22) Date of filing: 15.11.2024
(51) Int. Cl.: A61B 6/02, A61B 6/03, A61B 6/00

(54) **MOBILE X-RAY SYSTEM FOR 3D MEDICAL IMAGING OF A SUBJECT**

(71) Applicant: Koninklijke Philips N.V., 5656 AE Eindhoven (NL)
(72) Inventor: DE BOER, Jacob, Eindhoven (NL)
(74) Representative: Philips Intellectual Property & Standards

(57) **Abstract**

An X-ray system for medical imaging of a subject on a patient table, comprising a detector; an X-ray source; an arm support for carrying the detector and the X-ray source. The arm is controlled to follow a specific trajectory in order to acquire an enhanced 3D image of the subject.

## Description

### FIELD OF THE INVENTION

The invention relates to an X-ray system for medical imaging of a subject on a patient table, to a method of X-ray imaging in particular with an X-ray system and to computer program product for generating a 3D image of a subject.

### BACKGROUND OF THE INVENTION

Medical procedures such as surgeries require reliable 3D information of the region of interest of the subject (patient) to be diagnosed or treated.

Various solutions based on X-ray systems have been proposed to generate 3D images as 3D information of a subject. For example, WO2019063657 or WO20210011574 relate to C-arm type interventional X-ray imaging systems that are designed to generate 3D images of a patient during an intervention. These systems can be fixed or mobile imaging systems. A 3D image is generated during the intervention by positioning the C-arm at a certain position with respect to a table supporting the patient such that the X-ray source and the detector rotate synchronously around a so-called propelling axis and around the patient. For certain procedures, such as surgery procedures, a mobile system may be preferred than a fixed system, as for example the mobile system may provide more freedom to place the X-ray source and the detector in regions of interest of the patient that may be difficult to access otherwise. However, using mobile systems may have certain drawbacks compared to using fixed systems. In particular, mobile systems will typically be lighter than fixed systems, often leading to less stable and less precise 3D acquisitions when moving the C-arm which represents an important part of the weight of the mobile system. One way to solve this problem may be for example to limit the speed of the C-arm of the mobile system, thereby limiting as much as possible mechanical vibrations or perturbations during the 3D scan. However, such reduction of speed may obviously impact the workflow during the procedure. Unfortunately, this problem becomes even more important if/when the region of interest of the patient to be imaged is larger than the imaging field of view (FOV) provided by the imaging system, because one may then need to shift horizontally the C-arm to a new position covering a missed part of the region of interest. Thus, repeating in this way a relatively low speed 3D scan/acquisition (compared to a fixed system) to image in 3D the large region of interest may impact so much the workflow than the medical personnel may prefer to compromise by choosing the fixed system.

There may be a need for providing a mobile X-ray imaging medical system that overcome the foregoing problems.

### SUMMARY OF THE INVENTION

In view of the above, it is an object of the present invention to provide a mobile X-ray system for medical imaging of a subject on a patient table to meet at least said need.

These and other objects, which become apparent upon reading the following description, are solved by the subject matter of the independent claims.

In an aspect, the invention provides a mobile X-ray system for medical imaging of a subject on a patient table, comprising: a detector; an X-ray source; a control unit, an arm support assembly having first and second ends for carrying the detector and the X-ray source; a rotation unit configured to rotate the arm support assembly around a propeller axis, wherein the control unit is configured to rotate around the propeller axis and move in a horizontal direction the detector and the X-ray source with respect to the table such that the X-ray source and detector synchronously follow a predefined helical trajectory with respect to the table; and wherein the control unit is further configured to process X-ray images acquired along the trajectory to generate a helical 3D image of the subject.

Thus, it is particularly advantageous to perform a helical image acquisition with a mobile X-ray system as this combination provides a very good compromise amongst at least acceptable speed of acquisition, acceptable mechanical perturbations and acceptable imaged area coverage. In other words, the inventor has realized that it is surprisingly possible to obtain a 3D image, preferably a spatially extended 3D image, of reasonably good quality with a mobile X-ray system, without impacting the clinical workflow, if a helical scan and acquisition is performed with such specific X-ray system.

In embodiments, the arm support assembly has a U-shaped form, and comprises a first flat longitudinal element holding at the first end the detector and a second flat longitudinal element holding at the second end the X-ray source, wherein each flat longitudinal element comprises an actuator for moving the detector and the X-ray source linearly along the corresponding first and second flat longitudinal elements, wherein the control unit is configured to control each actuator and the rotation unit in such a way that the detector and the X-ray source synchronously follow the predefined helical trajectory by moving linearly said X-ray source and said detector along the first and second flat longitudinal elements and by rotating the U-shape arm support assembly.

Such an "U-shaped arm" or a "U-shaped form" or a "U-shaped arm" may be defined as a carrier having a first end portion, a second end portion and an third portion mechanically linking the first and second portions, the first end portion comprising a generally flat surface supporting the X-ray source and the second end portion comprising a generally flat surface supporting the detector (which may also be called receiver) in such a way that the X-ray source and the detector mostly face each other. In other words, the first and second end portions are mostly parallel to each other. Alternatively, they may also be more or less divergently or convergently tilted one to the other in a particular case. In a "U-shaped arm", at least one of said flat surfaces has a length (i.e. the dimension parallel to the main axis of the patient table when the end portion lies in a horizontal position) significantly higher than the length of the X-ray source and/or the detector. This end portion(s) can thus be seen as an elongated shape or an "extension" of an arm (if one compares with a C-arm) parallel to the patient table (when the end portion(s) lies in a horizontal position), so extending along the body of the patient. The third portion may be of any shape or design as long as it links the two end portions of the U-shaped arm. This intermediary portion may be for instance of a V-shape or U-shape or a W-shape or C-shape or curved shape.

When the X-ray source and the detector are configured to move synchronously along the flat surface of the end portions of the U-shaped arm by means of its respective actuators, it becomes possible to realize the horizontal component of the helical trajectory without actuating any other mechanical element of the system. In other words, the helical trajectory can be realized by operating the rotation unit and the two actuators only. As an advantage, a lower mass distribution of the tube and detector has less impact on the dynamical behavior of the system.

In embodiments, the mobile X-ray system comprises a horizontal movement unit configured to move the arm support assembly in a horizontal direction, wherein the control unit is configured to control the horizontal movement unit and the rotation unit in such a way that the detector and X-ray source synchronously follow the predefined helical trajectory by moving horizontally the detector and the X-ray source with respect to the table and by rotating the arm support assembly.

An advantage is that a synchronized movement of the tube and detector is controlled and secured by only one horizontal movement.

In embodiments, the horizontal movement of the X-ray source and the detector is performed by operating both the horizontal movement unit and the two actuators. In other words, the Xray-source and the detector follow synchronously the horizontal component of the helical trajectory by means of a linear displacement along the flat ends of the U-shaped arm, which itself undergoes a horizontal displacement.

In embodiments, the mobile X-ray system further comprises motorized wheels configured to move such system on the floor, wherein the control unit is configured to control the motorized wheels and the rotation unit in such a way that the detector and the X-ray source synchronously follow the helical trajectory by moving horizontally the system on the floor and rotating the arm support assembly.

An advantage is that the horizontal movement is done on the floor; there is no additional/extra horizontal movement required on the system.

When these embodiments presented above are combined, e.g. a linear translation of the X-ray source and the detector with respect to the flat ends of the U-shaped arm and of the U-shaped arm itself, together with a rotation of the U-shaped arm around the propelling axis, more free space above and or around the patient can be obtained, which may be necessary in certain procedures during the workflow.

In embodiments, the arm support assembly comprises a first arm for carrying the detector, a second arm for carrying the X-ray source, and wherein the first and second arms are independent of each other and controlled by two distinct motors.

In embodiments, the controller is configured to acquire images only when the arm support assembly reaches and moves at constant speed. This may improve the quality of the image acquisitions.

In embodiments, the system further comprises an angulation support configured to angulate the arm support assembly around a second rotation axis.

Embodiments of the invention may provide other advantages. For example, by moving the detector and the X-ray source in linear direction during a propeller rotation a longer 3D imaging run can be realized. Besides, the patient doesn't have to be moved during the 3D acquisition. Instead, both X-ray source and the detector move linearly over the patient during the propeller rotation (e.g. from remote to close position with respect to along the linear path of the U-shaped arm). The detector can be arranged advantageously closer to a subject, which may lead to an improved image quality.

The term mobile in the term mobile X-ray system defines an X-ray system that is configured to be moved on the floor. It therefore comprises a main stand supported on the floor by wheels or rolls in contact with the floor.

The term synchronously shall mean together at the same time. In particular, when the X-ray source and the detector move synchronously, both move at the same time such that their positions in the space with respect to each other do not change in time. In other words, the X-ray source and the detector always face each other.

The term subject is to be understood broadly in the present case and may comprise any humans and any animals; it is sometimes herein referred to a patient.

The term patient table is to be understood broadly in the present case and may relate to a structure configured to arrange a subject for medical imaging. A patient table is usually seen as a table having a generally or mostly top flat surface adapted to support a subject, although other configurations or designs of the table can be applicable to the current disclosure. The patient table may be fixed in the room or movable in the room. The patient table may comprise one or more actuators for manipulating a position of the patient table in the room and/or relative to an X-ray system that is used herein. The patient table may also be a so-called floating table, wherein a part of the table is fixed and another part of the patient table, the so-called patient carrier, can float in a horizontal plane.

The term detector is to be understood broadly in the present case and may relate to any X-ray detector configured to detect X-rays and to provide a corresponding (electrical) signal to a control unit of the system to process such signals, e.g. for imaging (display) and/or calculating purpose. Preferably the detector is a flat detector comprising essentially a low ratio between its base and height as aforementioned.

The term X-ray source is to be understood broadly in the present case and may relate to any source configured to emit X-rays.

The term control unit is to be understood broadly in the present case and may relate to any hardware and/or software unit configured to control mechanical components such as the detector and the X-ray source, and to process the data, such as signals provided by the detector, e.g. for imaging or calculating/analyzing purpose. The control unit may comprise a CPU, a PLC and an interface for data exchange. The control unit may be a single entity or distributed on a plurality of entities. The control may be further configured to control one or more drive trains used in the system (e.g. angulation support, rotation unit, horizontal movement unit, and/or actuator).

The term horizontal direction or axis means a direction or axis with an orientation parallel to patient table surface.

The term support is to be understood broadly and may relate to any structural element configured to carry a detector and an X-ray source. The support may be made of lightweight material, e.g. aluminum or carbon fiber. The support may comprise a lightweight structure, e.g. ribs, honeycomb design or the like. This may advantageously result in better movements. This may be advantageous as higher speeds can be realized for an U-shaped support. The support may comprise additional features such as an alignment laser, a collimator handle and a button (e.g. button for switching the alignment laser).

The term U-shaped is to be understood broadly and may preferably relate to a form that comprises a shape like the capital U. This shape may comprise at least two flat longitudinal elements (i.e. linear elements) that are separated on one end by a gap and connected to each other on the other end by a third element. The longitudinal element preferably relates to a linear element. The longitudinal element may preferably comprise at least one plane surface. A linear guide arranged at such a longitudinal element may provide a linear movement along the longitudinal element. The third element may be also a longitudinal element or any other form (e.g. curved). Contrary to the c-shaped form, it has rather an arched shape. The U-shaped form may be realized by a single entity or by a plurality of entities. The longitudinal elements comprise a length that is preferably larger than the length of the third element. The length of the longitudinal elements may comprise a length that is preferably larger than the size (e.g. width or length) of the detector or the X-ray source.

The terms U-shape(d) form, U-shape(d) form assembly, or U-shape(d) arm, U-shape(d) arm support assembly are considered to have the same meaning.

The term actuator, as used herein, relates to any manual and/or motorized device configured to provide a linear movement. Preferably the actuator is motorized. The actuator may comprise a ball screw drive, a belt drive, a pinion rack drive or a linear direct drive. The actuator may be supplemented by a linear guide or linear bearing. The actuator may comprise a gear configured to move the detector and the X-ray source synchronously. In other words, only one actuator may be sufficient and used to provide a linear movement of the X-ray source and the detector. In addition, or alternatively, a first actuator is configured to provide a linear movement of the detector, and a second actuator is configured to provide a linear movement of the X-ray source. This may be advantageous in terms of flexibility as the detector and the X-ray source can be moved independently from each other. This may enable just retracting the detector from a working of a clinician over the patient such that the clinician can immediately start with his work on the patient (e.g. surgery). However, during a 3D imaging run (combination of rotation movement and linear movement) the X-ray source and the detector may have to be moved synchronously. The actuator may further comprise a linear guide, e.g. a ball linear guide, slide linear guide, rolling elements linear guide. At least one actuator may be arranged at the elongated element of the arm support assembly or the first actuator and the second actuator may be arranged on the respective elongated elements of the arm support assembly. The arm support assembly, in particular the elongated elements of the u-shaped support may be extended by means of the linear guides, wherein the detector and X-ray source are arranged at least partially on the linear guides. Alternatively, the u-shaped support, and in particular the elongated elements of the arm support assembly may not be extended by the linear guides. In summary this may be advantageous as only the X-ray source and the detector must be moved and not the arm support assembly for arranging the X-ray source and the detector around the part of the subject to be imaged.

According to an embodiment, the X-ray system may further comprise a rotation unit configured to rotate the arm support assembly around a first rotation axis. The rotation axis is defined as a horizontal axis, which is further parallel to the linear direction of movement of X-ray source or detector or to the longitudinal axis of the patient when he/she lies on the table. The rotation unit may comprise an e-motor and a control unit. This may be advantageous as with the rotation of the arm support assembly also a so-called propeller rotation can be realized. A propeller rotation relates to a portion of, or one or more turns of the u-shaped support around the first rotation axis. The rotation movement can be advantageously used for 3D imaging. This may be advantageous in terms of image quality. The rotation movement may be used in combination with a horizontal movement of the arm support assembly and or the linear movement by means of the actuator as described above. This may advantageously lead to a so-called helical 3D image. With this helical path (helical 3D image), longer 3D images (data sets) can be created even with smaller detectors while the subject may stay advantageously stationary on the patient table. The rotation movement may have a constant speediness during the 3D imaging. This may be advantageous in terms of accuracy as there is no acceleration and deceleration during the imaging process. Alternatively, the speediness may be varied during the imaging process to have higher radiation at location of the patient where higher resolution is needed and/or to acquire less or more images corresponding to a desired quality for the 3D helical image.

According to an embodiment, the mobile X-ray system may further comprise an angulation support configured to angulate the arm support assembly around a second rotation axis. The term angulation, as used herein, relates to a rotation of the arm support assembly along an arched guide rail (i.e. sleeve) around the second rotation axis. It should be noted that this rotation is limited by the dimensions of the sleeve. The second rotation axis depends on the geometry of the sleeve, in particular from an arch angle of the sleeve. The angulation support may comprise a sleeve configured to guide the arm support assembly. The angulation support may be arranged at the stand of the system. The angulation support may comprise an e-motor and a control unit configured to control the angulation support for angulating the u-shaped support. The angulation support may comprise additionally an extension sleeve configured to provide an extended angulation of the arm support assembly. The term extension sleeve, as used herein, relates to an arched guide rail configured to be mounted to the sleeve for extending an angulation degree. The angulation movement may be advantageously combined with the rotation movement of the arm support assembly, and/or the linear movement of the detector and the X-ray source and/or the horizontal movement of the arm support assembly during an imaging process.

A further aspect of the present disclosure relates to a computer program product comprising computer-readable instructions, which, when executed on a controller, cause the controller to control the mobile X-ray system of the invention by
- rotating around a propeller axis and moving in the horizontal direction the detector and the X-ray source with respect to the table such that the X-ray source and detector synchronously follow the predefined helical trajectory,
- acquiring X-ray images during the trajectory, and
- processing the X-ray images to generate a helical 3D image.

The computer program product may further cause the controller to control the mobile X-ray system by controlling each actuator and the rotation unit in such a way that the detector and the X-ray source follow the predefined helical trajectory by moving linearly along the first and second flat longitudinal elements the X-ray source and the detector, respectively, and by rotating the U-shape arm support assembly.

The computer program product may cause the controller to control the mobile X-ray system by controlling the horizontal movement unit and the rotation unit in such a way that the detector and X-ray source follow the predefined helical trajectory by moving horizontally the detector and the X-ray source with respect to the table while synchronously rotating the arm support assembly.

The computer program product may cause the controller to control the mobile X-ray system by controlling the motorized wheels and the rotation unit in such a way that the detector and the X-ray source follow the helical trajectory by moving horizontally the system on the floor while synchronously rotating the arm support assembly.

A further aspect of the present disclosure relates to a method of generating a 3D helical image with the system of the invention. The method comprises the steps of rotating around a propeller axis and moving in the horizontal direction the detector and the X-ray source with respect to the table such that the X-ray source and detector synchronously follow the predefined helical trajectory, and acquiring X-ray images during the trajectory, and processing the X-ray images to generate a helical 3D image.

It is noted that the above embodiments may be combined with each other irrespective of the aspects involved.

These and other aspects of the present invention will become apparent from and elucidated with reference to the embodiments described hereinafter.

### BRIEF DESCRIPTION OF THE DRAWINGS

Exemplary embodiments of the invention will be described in the following drawings.
Fig. 1A shows a schematic view of a mobile X-ray system for medical imaging of a subject on a patient table in a first linear position of the tube/detector, wherein the arm support arm assembly has a U-shape and wherein the X-ray-source and the detector can move linearly along the flat portions of the U-shape arm support assembly;
Fig. 1B shows a schematic view of the mobile X-ray system of Fig. 1A together with a schematic illustration of a helical trajectory that the X-ray tube and the detector may follow in accordance with the principles of the invention;
Fig. 2 shows a schematic view of the mobile X-ray system, illustrating that the horizontal component of the helical trajectory may be obtained by moving the table horizontally; and
Fig. 3 shows a schematic view of the mobile X-ray system, illustrating that the horizontal component of the helical trajectory may be obtained by moving motorized wheels on the floor; and
Fig. 4 shows a flow chart of a method for medical imaging with a mobile X-ray system according to the invention.

### DETAILED DESCRIPTION OF THE EMBODIMENTS

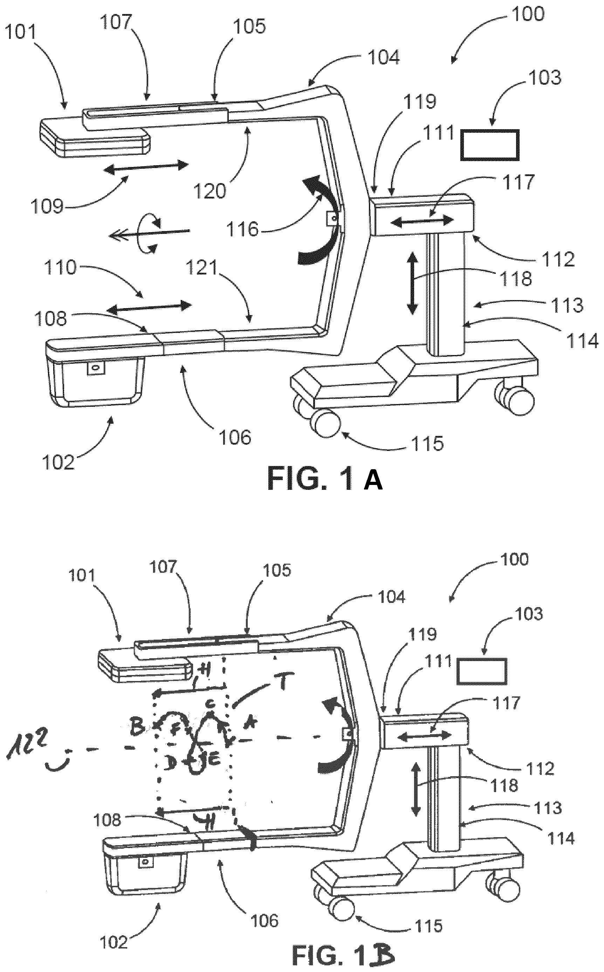

Fig. 1A shows a schematic view of a mobile X-ray system 100 for medical imaging of a subject on a patient table in a first position. The mobile X-ray system is in the present example a mobile CBCT system. The mobile X-ray system comprises a detector 101 and an X-ray source 102. The detector 101 is in the present example a flat detector. The system 100 further comprises a control unit 103. Control unit 103 is configured to control movement of the detector 101 and the X-ray source 102 via first actuator 107 and second actuator 108. The control unit 103 is depicted schematically adjacent to or independent from the structural elements of the system, for instance included in a mobile stand control unit being a tower (including the circuitry and algorithms) and a display (e.g. a screen) or for instance in (mobile) compute, however it will be clear to the skilled person that the control unit 103 can also be integrated in the structural element of the system, for example in the stand 114. System 100 further comprises an arm support assembly 104 for carrying the detector 101 and the X-ray source 102. The arm support assembly 104 is an arm that has a U-shaped form in this embodiment. The arm support assembly 104 comprises a first flat longitudinal element 120 and second flat longitudinal element 121. The arm support assembly 104 is arranged at stand 114. Detector 101 is arranged at a first end 105 of the first flat longitudinal element 120. The X-ray source 102 is arranged at a second end 106 of the second flat longitudinal element 121. System 100 comprises a first actuator 107, which is at least partly arranged at the first end 105 of the first flat longitudinal element 120. The first actuator 107 is further mechanically connected with detector 101. The first actuator 107 is configured to provide a linear movement 109 of detector 101 along the flat longitudinal element 120. The first actuator 107 may comprise, as an exemplary embodiment, a ball screw spindle and a linear guide. The first actuator may alternatively comprise one of the following: a belt drive, a pinion rack drive or a linear direct drive. This enables system 100 to increase the positioning capability of the detector 101. The system 100 comprises further a second actuator 108, which is at least partly arranged at the second end 106 of the second flat longitudinal element 121. The second actuator 108 is further mechanically connected with the X-ray source 102. The second actuator 108 is configured to provide a linear movement 110 of the X-ray source 102 along the flat longitudinal element 121.

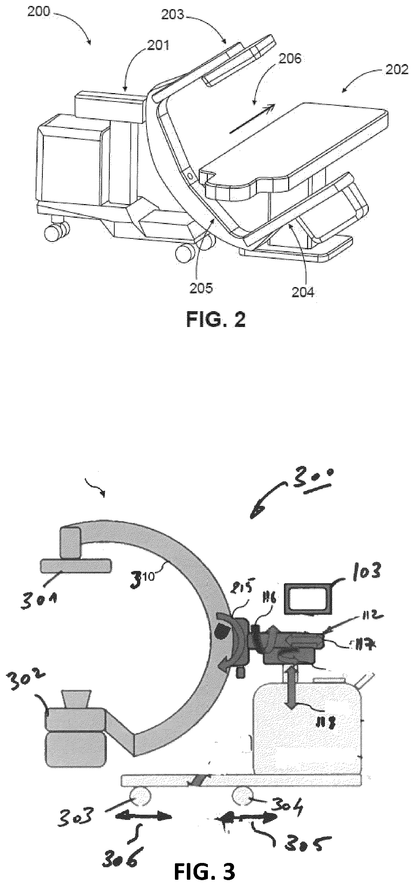

The linear movements are along the ends of the flat longitudinal elements, in the direction depicted by the arrows 109 and 110 in Fig. 1A. As the U-shaped arm assembly may be angled around the second rotation axis 206 (see Fig. 2) such that the flat longitudinal elements (and therefore the X-ray source and detector image planes) lie in the horizontal direction parallel to the table, the linear movements long the ends of the flat longitudinal elements are also horizontal. The second actuator 108 may comprise, as an exemplary embodiment, a ball screw spindle and a linear guide. This enables the system 100 to increase the positioning capability of the X-ray source 102. In the present example the actuators 107 and 108 are extended, which means that the X-ray source and the detector are also extended such that they could reach e.g. further over a patient table.

System 100 comprises in the present example further a rotation unit 111. The rotation unit 111 is arranged in stand 114, for example integrated in a yoke, as well-known. The rotation unit 111 is configured to rotate the u-shaped support 104 around a first rotation axis 122 which in the present of example lie along a horizontal axis. The rotation movement 116 can be carried out in both directions. This rotation movement 116 is called propeller rotation and is not limited to a certain degree. System 100 comprises in the present example a horizontal movement unit 112 as well-known. The horizontal movement unit 112 is arranged at the stand 114, wherein the horizontal movement unit 112 is configured to move the u-shaped support 104 in a horizontal direction 117. System 100 comprises in the present example further wheels 115 and is therefore movable. Hence it is possible for staff to move the system 100 for a rough positioning. In embodiments, the wheels are motorized independently or not, and in other embodiments the wheels are not motorized (free). System 100 comprises in the present example further a lifting unit 113 configured to lift the U-shaped support 104 in a vertical direction 118. The lifting unit 113 may be arranged in stand 114. System 100 comprises in the present example further an angulation support 119 configured to angulate the U-shaped support 104 around a second rotation axis (illustrated in Fig. 2) as well-known. The angulation support 119 may be arranged in stand 114. The angulation support 119 provides an angulation movement of the U-shaped support 104 around a second rotation axis. The control unit 103 is in the present example further configured to provide control signals for controlling the actuators 107 and 108, the rotation unit 111, the lifting unit 113 the horizontal movement unit 112 and the angulation support 119. Alternatively, those movements can be implemented manually only, or manually with the assistance of motors driving the respective propelling, horizontal, vertical and/or angular movements. Such motor(s) may be integrated within the stand. Furthermore, some braking systems (such as e.g. EM brakes) could be further used within the motorized and/or manual arrangement of the system, together with associated controlling tool, to slow-down the movements for accuracy or safety positioning of the system. The U-shaped arm may be arranged with linear drivetrains. With this U-shaped arm the internal depth can be increased towards 1200 mm and even more so it can shift over the patient and the patient table. The U-shaped arm can make a plurality of turns for 3D imaging with higher speeds and combined with the linear movements of the X-ray source and X-ray detector to create larger 3D volumes (helical 3D volumes), the perfectly balanced U-shaped arm can rotate with higher speeds, which makes it useful for multi applications.

Fig. 2 shows a schematic view of the same system 100, here designated as system 200. In contrast to Fig. 1 the actuators 203 and 204 are in a retracted position. Indeed, the actuators have been translated from the position of Fig. 1A at a distance away from the stand or the center of the middle portion of the U-shaped support, to a new position which is here as near as possible to said center of the middle portion. Fig. 2 also illustrates embodiments of the system of the invention that can be combined with any other embodiments and characterized by an angulation support 201 mounted on the stand for angulating the U-shaped support 205 around a second rotation axis 206 perpendicular to the first rotation axis 122.

Fig. 1B shows the system of Fig. 1A and illustrates an example of a helical trajectory T that the U-shaped support may follow in accordance with the principles of the invention. More precisely, the control unit 103 may operate the system to combine a rotation of the U-shaped support 104 around the first rotation axis 122 and a translation in the horizontal direction (H) of the X-ray source 102 and detector 101, in such a way that the X-ray source and detector synchronously follow a predefined helical trajectory T with respect to a table placed in the space defined in the U-shaped arm. In this example the helical trajectory T is making more than one full rotation around the first rotation axis 122 between a start point A and an end point B of the trajectory scanned. In this example, the trajectory goes in a forward direction (as indicated by the arrow on trajectory T in Fig. 1B), namely the start point A is closer to the stand 112 and the end point B is at a further distance away from the stand 112. In other examples the scan along the trajectory T may go in the opposite direction, that is in a backward direction. In embodiments the helical trajectory T is realized by having the control unit operating the actuators 107 and 108. In this way the detector 101 and the Xray-source 102 follow the linear translation component of the helical trajectory from start point A to end point B. At the same time, the control unit operates the rotation unit to cause the detector 101 and the Xray-source 102 follow the propelling component of the helical trajectory from start point A to point end point B.

In embodiments, the helical trajectory is followed by having the control unit operating the horizontal movement unit 112. In this way the detector 101 and the Xray-source 102 follow the linear translation component of the helical trajectory from start point A to end point B. At the same time, the control unit operates the rotation unit to cause the detector 101 and the Xray-source 102 follow the propelling component of the helical trajectory from start point A to end point B.

In embodiments the helical trajectory is followed by having the control unit operating the wheels 115, which should be motorized such as case. In this way detector 101 and the Xray-source 102 follow the linear translation component of the helical trajectory from start point A to end point B. At the same time, the control unit operates the rotation unit to have the detector 101 and the Xray-source 102 follow the rotation (propelling) component of the helical trajectory from start point A to end point B. It may also be interesting to follow the linear translation component of the helical trajectory from start point A to end point B by combining these embodiments. In particular, it may be interesting to command the horizontal movement unit 112 as well as the actuators 107 and 108 during a scan, and at the same time or not. It may therefore be possible to obtain an even more extended length of the helical scan, which can for example be interesting for spine procedures or for a total scan of a leg. It may also be interesting to command the motorized wheels as well as the actuators during a scan, and at the same time or not.

In embodiments, the scan may be performed in a continuous manner, namely without interruption of the movement along the helical trajectory. The speed of image acquisition during the helical movement may be fixed (constant) but it may differ from one scan to another. It may be advantageous to predefine in a memory of the system the different parameters of speed for the different clinical applications. Low speeds (e.g. in the order of 15 degrees/seconds) may be beneficial for a better dynamical behavior and/or for generating more images for a better image quality (IQ). This may be of interest when imaging bones, for example. High speeds (e.g. in the order of 90 degrees/seconds) may be required for critical situations like for neuro scans after brain damages or for cardiac scans often performed in combination with contrast fluids. Higher speeds may be of interest for imaging tumor or neuro related applications for example.

In embodiments, the dynamic mechanical behavior of the arm support assembly may be taken into account to define a time to start the acquisition of images and a time to stop the acquisition, both different from the times at which the movement of the arm support assembly starts and ends. For example, the acquisition of images may start only after the arm support assembly has accelerated and reached a constant speed and only until or before it decelerates until stopping its movement. Such dynamic behavior of the arm support assembly may be taken into account by predefining and storing in the memory of the system two moments in time that the person skilled in the art finds appropriate in view of the knowledge of the mechanical behavior of the system. Alternatively, these two moments in time may be automatically determined by the system by analyzing the actual speed of the arm support assembly in movement by means of at least one sensor integrated to the system (e.g. a speed sensor, accelerometer sensor, etc.). As an advantage, a better dynamical behavior can be obtained as well as an improved image quality.

In embodiments, the scan may be performed with certain predefined interruptions of the movement. For example, an image may be acquired at start point A, the actuators 107 and 108, and the rotation unit may then be operated to move and stop the detector and the X-ray source at different points along the helical trajectory T, for example at stop points C, D, E, F and B, to acquire in total six corresponding images that may be combined (for example stitched together) to generate a 3D helical image of the patient.

In all the embodiments of the invention, control unit 103 receives the images from the detector and processes these images to create a 3D image according to techniques well-known in the art. In particular these images can be combined or stitched together in accordance to methods known in the art.

The generation of this 3D image may be performed once all images expected from the helical scan have been acquired or on the fly (while any new image is obtained during the helical scan).

Embodiments based on the U-shape arm support assembly is also a kind of an mobile X-ray system with specific moving ranges over the patient at the end of the table. This makes it possible to make 3D images of the patient reaching out to the complete spine and pelvis. By combining the U-shaped support with embodiments presented herewith that use the actuators to extend even further the length of the first and second U-shaped support portions, even longer 3D helical image volumes can be obtained. Multi turns and linear movements can be combined during one 3D imaging run. The longer U-shaped support may also offer the possibility to reach further over the patient, when the U-shaped support is positioned at one of the end positions of the patient table for performing a 3D scan. The U-shaped support is able to obtain a 3D helical image for example from a part of the patient arranged on the middle of the patient table to the end position of the patient table that is closer to the stand of the system. In other words, the embodiments based on U-shaped support mobile systems also enable to provide a helical trajectory that can extend to regions of interest that may not be reachable with some other type of arms (e.g. C-arm).

Fig. 3 shows a simplified schematic of a mobile X-ray system 300 comprising a C-arm 301 as an arm support assembly. Reference numerals that are provided similar to the previously presented Figures designate the same elements of the mobile X-ray system. A detector 301 and an X-ray source 302 are mounted at the extremities of the C-arm. The C-arm is supported by the stand, for example like in the system of Fig. 1A, and the stand is supported by three or more motorized wheels (two of them being represented by references 303 and 304). Control unit 103 commands the wheels and the rotation unit to realize a helical trajectory that should be followed by the detector and the X-ray source synchronously to obtain a 3D helical image. Arrows 305 and 306 in Fig. 3 illustrate the horizontal directions that the system may follow during a helical scan by operating the motorized wheels accordingly. These directions are parallel to the first axis of rotation and may be parallel or perpendicular to longest axis (dimension) of the table.

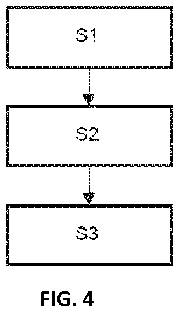

Fig. 4 shows a flow chart of a method for medical imaging with a mobile X-ray system.

The method of X-ray imaging is used in the present example with a mobile X-ray system described above. The X-ray system therefore comprises as an example a support, an X-ray source and a detector. The support may have U-shaped form, wherein the U-shaped form comprises a first flat longitudinal element and a second flat longitudinal element, wherein the detector is arranged at a first end of the first flat longitudinal element and the X-ray source is arranged at a second end of the second flat longitudinal element. The method comprises the following steps: The first step comprises linearly moving and rotating the X-ray source and the detector such that they synchronously follow a predefined helical trajectory with respect to the table (step S1). The second step comprises acquiring X-ray images during the helical trajectory (step S2). The third step comprises creating a 3D helical image by combining these images (step S3).

In an example, not further shown in detail, a computer program is provided enabling a controller or processor to carry out the method as described herein above.

In an example, a computer program or program element for controlling a mobile system as described herein above is provided, which program or program element, when being executed by a processing unit, is adapted to perform the method steps of one of the method examples above.

In an example, not further shown in detail, a computer readable medium is provided having stored the program element of claim of the examples above.

The computer program element might be stored on a computer unit or be distributed over more than one computer units, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer, as notably presented herein-before. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors or controllers.

While the invention has been illustrated and described in detail in the drawings and foregoing description, such illustration and description are to be considered illustrative or exemplary and not restrictive. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor, controller or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A mobile X-ray system for medical imaging of a subject on a patient table, comprising:
- a detector;
- an X-ray source;
- a control unit,
- an arm support assembly having first and second ends for carrying the detector and the X-ray source;
- a rotation unit configured to rotate the arm support assembly around a propeller axis,
wherein the control unit is configured to rotate around the propeller axis and move in a horizontal direction the detector and the X-ray source with respect to the table such that the X-ray source and the detector synchronously follow a predefined helical trajectory with respect to the table; and
wherein the control unit is further configured to process X-ray images acquired along the trajectory to generate a helical 3D image of the subject.

2. System according to claim 1, wherein the arm support assembly has a U-shape form and comprises a first flat longitudinal element holding at the first end the detector and a second flat longitudinal element holding at the second end the X-ray source, wherein each flat longitudinal element comprises an actuator for moving the detector and the X-ray source linearly along the corresponding first and second flat longitudinal elements, wherein the control unit is configured to control the actuator and the rotation unit in such a way that the detector and the X-ray source synchronously follow the predefined helical trajectory by moving linearly said X-ray source and said detector along the first and second flat longitudinal elements and rotating the U-shape arm support assembly.

3. System according to claim 1 or 2, further comprising a horizontal movement unit configured to move the arm support assembly in a horizontal direction, wherein the control unit is configured to control the horizontal movement unit and the rotation unit in such a way that the detector and X-ray source synchronously follow the predefined helical trajectory by moving horizontally the detector and the X-ray source with respect to the table and rotating the arm support assembly.

4. System according to claim 1, further comprising motorized wheels configured to move the system on the floor, wherein the control unit is configured to control the motorized wheels and the rotation unit in such a way that the detector and the X-ray source synchronously follow the helical trajectory by moving horizontally the system on the floor and rotating the arm support assembly.

5. System according to any of the preceding claims, wherein the arm support assembly comprises a first arm for carrying the detector, a second arm for carrying the X-ray source, and wherein the first and second arms are independent of each other and controlled by two distinct motors.

6. System according to any of the preceding claims, wherein the controller is configured to acquire images, only when the arm support assembly moves at constant speed.

7. The system according to any one of the preceding claims, further comprising an angulation support configured to angulate the arm support assembly around a second rotation axis.

8. A computer program product comprising computer-readable instructions, which, when executed on a controller, cause the controller to control the mobile X-ray system of claim 1 by
- rotating around a propeller axis and moving in the horizontal direction the detector and the X-ray source with respect to the table such that the X-ray source and detector synchronously follow the predefined helical trajectory,
- acquiring X-ray images during the trajectory, and
- processing the X-ray images to generate a helical 3D image.

9. A computer program product according to claim 8, which, when executed on the controller, cause the controller to control the mobile X-ray system of claim 2 by controlling each actuator and the rotation unit in such a way that the detector and the X-ray source follow the predefined helical trajectory by moving linearly along the first and second flat longitudinal elements the X-ray source and the detector, respectively, and by rotating the U-shape arm support assembly.

10. A computer program product according to claim 8, which, when executed on the controller, cause the controller to control the mobile X-ray system of claim 2 or 3 by controlling the horizontal movement unit and the rotation unit in such a way that the detector and X-ray source follow the predefined helical trajectory by moving horizontally the detector and the X-ray source with respect to the table while synchronously rotating the arm support assembly.

11. A computer program product according to claim 8, which, when executed on the controller, cause the controller to control the mobile X-ray system of claim 4 by controlling the motorized wheels and the rotation unit in such a way that the detector and the X-ray source follow the helical trajectory by moving horizontally the system on the floor while synchronously rotating the arm support assembly.

12. Method of X-ray imaging with a mobile X-ray system according to any of claims 1 to 7, the method comprising the following steps:
- linearly moving and rotating the X-ray source and the detector such that they synchronously follow a predefined helical trajectory with respect to the table (S 1),
- acquiring X-ray images during the helical trajectory (S2),
- creating a 3D helical image by combining the acquired images (S3).
